(19) Europäisches Patentamt European Patent Office Office européen des brevets

(11) **EP 2 021 764 B1**

(12) **EUROPEAN PATENT SPECIFICATION**

(45) Date of publication and mention of the grant of the patent:
**04.04.2018 Bulletin 2018/14**

(21) Application number: **07728433.9**

(22) Date of filing: **24.04.2007**

(51) Int Cl.:
**G01N 15/05** (2006.01)    **G01N 33/49** (2006.01)

(86) International application number:
**PCT/EP2007/053977**

(87) International publication number:
**WO 2007/128684 (15.11.2007 Gazette 2007/46)**

(54) **METHOD TO DETECT STATES OF ANEMIA PRESENT IN A BLOOD SAMPLE**

VERFAHREN ZUM NACHWEIS VON IN EINER BLUTPROBE ANWESENDEN ANÄMISCHEN ZUSTÄNDEN

PROCEDE POUR DETECTER DES ETATS D'ANEMIE DANS UN PRELEVEMENT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.04.2006 IT UD20060111**

(43) Date of publication of application:
**11.02.2009 Bulletin 2009/07**

(73) Proprietor: **Alifax S.r.L.**
**35020 Polverara (PD) (IT)**

(72) Inventors:
• **GALIANO, Paolo**
  **I-35100 Padova (IT)**
• **CIOTTI, Alfredo**
  **I-33100 Udine (IT)**

(74) Representative: **Petraz, Gilberto Luigi et al**
**GLP S.r.l.**
**Viale Europa Unita, 171**
**33100 Udine (IT)**

(56) References cited:
**EP-A- 0 638 799**    **EP-A- 1 356 859**
**EP-A2- 1 098 188**    **US-A- 3 549 994**
**US-A- 4 352 557**

• **J G G Dobbe: "Engineering developments in hemorheology", , 19 September 2002 (2002-09-19), XP055365158, Retrieved from the Internet: URL:https://pure.uva.nl/ws/files/3504357/2 2841_UBA002000669_06.pdf [retrieved on 2017-04-18]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention concerns a method to detect states of anemia present in a blood sample. To be more exact, the invention allows to detect states of anemia simultaneously with the clinical test to detect the erythrocyte sedimentation rate, or ESR, substantially using the same apparatus and the same method.

BACKGROUND OF THE INVENTION

It is known that anemia is a physiological condition that can be detected in a blood sample when the quantity of hemoglobin goes below 13 grams % in men and 12 grams % in women, irrespective of whether the number of red corpuscles can go below 4.5-5 million erythrocytes per $mm^3$ (oligocythemia).

The ratio between plasma (liquid component of the blood) and the corpusculate part constitutes the hematocrit or Htc. This normally must be comprised between 40-45% in men and between 37-47% in women. Normal erythrocytes, or red corpuscles, have the shape of a biconcave lens, with a diameter that oscillates between 6.7-7.7 micron and a thickness of 2 micron. They contain a corpuscular hemoglobin concentration equal to 27-32 pg (picograms); this diminishes, for example, in thalassemia and microcytic hyposiderimic anemia (that is, with small red corpuscles and with low Hb). Corpuscular hemoglobin is calculated from the ratio Hb%/red corpuscles in millions/$mm^3$.

Hemoglobin (Hb) consists of a protein part (globine) and the prosthetic group of the heme, which contains tetrapyrrolic nuclei with the iron at the center of the structure, the function of which is to "oxidize", to transport the oxygen and to give it up to the tissues. There are numerous classifications used to catalog anemias: one of these takes into consideration the MCV, or mean corpuscular volume, which is equal to the ratio between the hematocrit and the red corpuscles (r.c.) in millions x $mm^3$ and the corpuscular value = Hb%/number of r.c. x 20, and is among the most followed.

We will therefore have the following conditions:

-   normochromic anemia, with a corpuscular value equal to 1;
-   hyperchromic anemia, with a corpuscular value > 1;
-   hypochromic anemia, with a corpuscular value < 1.

Moreover, another criterion classifies anemias according to the MCV and the MCHC (mean corpuscular hemoglobin concentration = Hb%/Hct (hematocrit));

-   hypochromic microcytic anemia (that is, normal content in Hb per r.c. + r.c. of decreased sizes, for example thalassemia and hyposiderimic anemia);
-   normochromic normocytic anemia (that is, normal content in Hb per r.c. + normal sizes, therefore post-hemorrhagic anemia);
-   hypochromic macrocytic anemia (that is, r.c. of greater size + higher Hb content, for example in B12 deficiencies and folates, because the r.c. is not able to have a normal erythropoiesis due to lack of vitamins).

Other classifications of anemia can be the following:

On a pathogenetic basis:

-   anemia due to production deficit;
-   anemia due to excess consumption;

on an anatomic-functional basis:

-   normogenerative anemia (with normoreticulocytosis);
-   hyporegenerative anemia (with hyporeticulocytosis);
-   hyper-regenerative anemia (with hyper-reticulocytosis);

based on signs of hyper-haemolysis:

-   hemolytic anemia
-   anhemolytic anemia.

Another classification is a biochemical one, according to which we distinguish:

- hyposiderimic anemia (proper iron deficiency, and iron deficiency anemia as a congenital defect of the mobilization of the iron);
- hypersiderimic anemia (caused by a defect in the use of the iron - sidero-acrestic);
- aplastic anemia, caused by the excessive destruction of the red corpuscles (thalassemia, hemolytic anemia in general);
- morphological anemia (very followed, see above);
- normocytic anemia (that is, normal volume);
- macrocytic anemia (that is, with red corpuscles of increased volume, with an mean corpuscular volume (MCV) $\geq 95$ micron$^3$;
- microcytic anemia (that is, red corpuscles with an MCV $< 76$ micron$^3$; and therefore due to the concentration of hemoglobin (Hb) in every red corpuscle:
- normochromic anemia (hence with a normal concentration of hemoglobin per corpuscle of 27 picograms);
- hyperchromic anemia (with an increased concentration of Hb);
- hypochromic anemia (hence with a decreased concentration of Hb).

In any case, anemia can substantially be divided into:

I) Anemia caused by the decreased production of red corpuscles;
II) Anemia caused by increased destruction;
III) Anemia dueto loss.

On the contrary, polycythemia causes an increase in the production of red corpuscles and hence an increase in the hematocrit.

Anemia is not a direct cause of a high value of ESR, as much as the fact that the red corpuscles, as they aggregate, form rouleaux and therefore, as described by Stokes, cause sedimentation thereof with increased speed, a phenomenon which is indirectly visible with the known, Westergren method.

With reference to the Westergren method, literature has described types of anemia where the ESR is increased, others where it is absent. For example, ESR is increased in megaloblastic anemia rather than in sideropenic anemia. On the contrary, a high polycythemia inhibits sedimentation.

In the same way, great modifications to the shape of the red corpuscles inhibit their sedimentation, such as for example spherocytic anemia, falciform anemia, and also sedimentation is decreased in thalassemic microcytemia. From the above it can be concluded that the presence of anemia does not necessarily mean increased values of ESR. It is evident that the red corpuscles, subjected to the force of gravity, even without aggregation, will sediment, but at a speed that the Westergren method will not be able to appreciate.

It is known, from EP-A-1.090.188 (EP'188) an apparatus and a method to determine the speed of sedimentation of blood (ESR) by detecting the development over time of the optical density, or absorbance, of a sample of blood when subjected to a sudden interruption of the flow of the blood sample ("stop-flow" effect), obtaining the break-up of the rouleaux and the subsequent re-aggregation and formation of the rouleaux.
It is further known that the transmittance curve, in a known manner, as described for example in US-A-4.352.557, is represented by the syllectogram shown in the attached fig. 2.

It is also known from EP-A-0.638.799 (EP'799) that an increased aggregability of the red blood cells occurs in many disease states (e.g. coronary heart diseases, diabetes, hyperviscosity syndrome, thrombosis, trauma and sickle cell anemia) and is considered to be a risk factor. EP'799 discloses an apparatus that enables the visualization of red blood cells in a flow cell and analysis of the aggregability size and deformability under varying flow conditions so as to monitor the cell-to-cell interaction which is altered in pathological states and affected by drugs.

The apparatus of EP'799 provides for a flow of a cell suspension in a reservoir, which can be developed in a repeatable way by switching between a pushing and a pulling operation determined by a supply syringe so as to obtain a prolonged detection of cell suspension, wherein the detection is carried out, in a real-time manner, by a microscope and a video camera connected each other and a computer with an image monitor connected thereto.

Based on all these considerations explained above, the purpose of the present invention is to obtain a detection, as objective and rigorous as possible, of the states of anemia in a blood sample using, in a novel way and basing the detection on a surprising intuition of the Applicant, the information deducible from the detection of the ESR particularly at the moment when the break-up of the rouleaux which have possibly formed is provoked. This break-up of the rouleaux can be obtained, for example but not only, by using the method and apparatus described in the European patent application EP-A-1.098.188 (EP'188), in the name of the present Applicant; or the method which uses the cell system with agitator as described in US-A-4.352.557; or the method which uses a vibration system, able to be selectively activated and associated with a blood sample container, or more generally any method that determines, after a transitory step of aggregation of the rouleaux, due to a state of rest, a break-up due to an instantaneous interruption of the state of rest.

The Applicant has devised, tested and embodied the present invention to obtain this purpose and other advantages as will be shown hereafter.

SUMMARY OF THE INVENTION

The present invention is set forth and characterized essentially in the main claim, while the dependent claims describe other innovative characteristics of the invention.

In the description that follows, we shall mainly refer, as applicative method, to the apparatus for the optical detection of the optical absorbance described in the above-mentioned EP-A-1.098.188; the invention, as we said, can be used employing other systems to interrupt the state of rest of a blood sample, for example the cell system as described in US'557, or others again.

To be more exact, fig. 1 shows a photometer usable in the method according to the present invention, fig. 2 a syllectrometric diagram, fig. 3 a diagram of the anemia factor and fig. 4 a diagram of the photometric hematocrit.

The Applicant has surprisingly deducted that the method and the apparatus described in EP'188, and the other methods above mentioned, allow to evaluate quickly, simply and reproducibly, not only a value of aggregation of the corpuscular part of the blood, from which the ESR value expressed in mm/h derives, as widely disclosed in the known literature, but also a value of "difficulty of aggregation" of the red corpuscles (difficulty in forming rouleaux). These values, with a very high probability, are representative and connectable to pathologies, and particularly to a state of anemia, as disclosed hereinbelow, present in the person from whom the blood sample was taken.

For a greater understanding of the phenomenon, reference should be made to the following table, relating to the main characteristics of the corpuscular part of the blood, from which it can be deduced that on average about 98% of the corpuscular part is represented by the red corpuscles, while the remaining 2% by the other cells.

Philosophy of the Anemia Factor

| Cell | Number of cells x mm$^3$ | Shape and size | Volumetric concentration % |
|---|---|---|---|
| Erythrocytes | 4-6 x 10$^6$ | Biconcave disks | 45 |
| Total Leucocytes<br>Granulocytes Neutrophiles<br>Eosinophiles<br>Basophiles<br>Lymphocytes<br>Monocytes<br>Platelets | 4-11 x 10$^3$<br>1.5-7.5 x 10$^3$<br>0-4 x 10$^2$<br>0-2 x 10$^2$<br>1-4.5 x 10$^3$<br>0-8 x 10$^2$<br>250-500 x 10$^3$ | Roughly spherical<br><br><br><br><br><br>Rounded or oval | 1 |

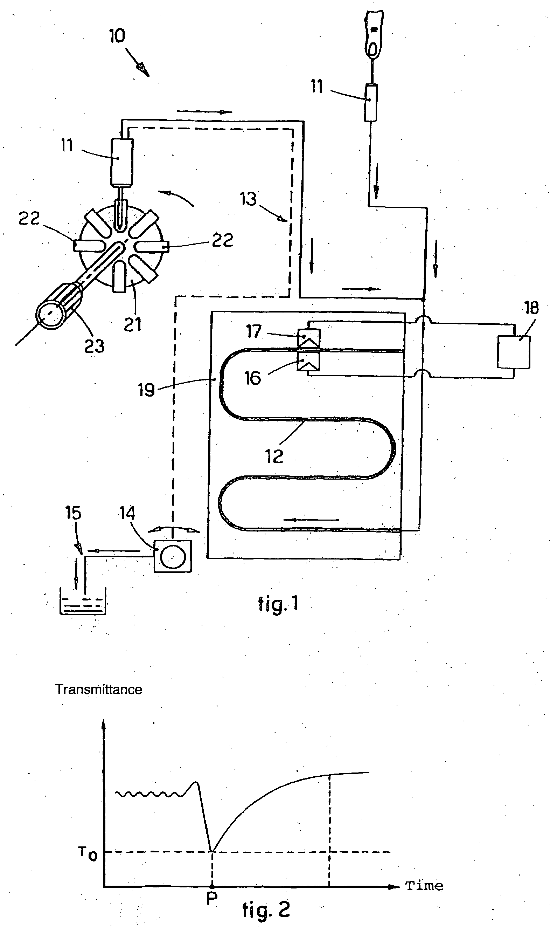

362 blood samples were measured, photometrically evaluating the density of the whole blood, in this specific case using the photometer of the apparatus described in EP'188 (fig. 1), which uses an optical path of 0.8 mm.

In short, the apparatus 10 comprises a capillary tube 12 through which a blood sample is made to flow, picked up by a pick-up member 11 which acts directly on a part of the human body, or on a collector 21, where a plurality of test tubes 22 are contained, able to be selected by activating a motor 23.

Along the capillary tube 12 an optical detection system is provided, comprising a light emitter 16 and a mating receiver 17. A pumping member 14 is also associated with the capillary tube 12, which causes the blood sample to be picked up and pass through the capillary tube 12; it also causes the instantaneous interruption of the flow, in order to cause the aggregation and formation of the rouleaux. Downstream of the pumping member 14 there is a discharge pipe 15 provided for the blood sample.

The apparatus 10 also comprises a command and control unit 18 which manages the functioning of the apparatus 10 and processes the information acquired, constructing the transmittance curve of the blood sample after the interruption of the blood flow and the formation of the rouleaux.

The transmittance curve, in a known manner, as described for example in the above-mentioned US'557, is represented by the syllectogram shown in fig. 2, where the temporal point, or instant, P represents the instant of interruption of the blood flow, or the moment of maximum break-up of the rouleaux which formed during the normal, unobstructed flow. In this temporal instant P the measured transmittance reaches its minimum value $T_0$.

In terms of absorbance, for the 362 samples values of $A_0$ were measured that vary from 0.5 to 0.95. The main cause of absorbance of light in the photometer is determined by the presence of the red corpuscles, cellular elements that, as we saw above, constitute the main component (98% - see Table 1) of the corpusculate part and the density of which is determined by the quantity of hemoglobin individually present.

In order to obtain a correlation and check factor between the values of the syllectogram and the state of anemia of the patients subjected to measurement, a hemochrome examination was done on the same 362 samples of blood, in this specific case using the Coulter MAXM corpuscle-counter, to determine the main parameters of the blood, such as, white corpuscles (WC), red corpuscles (RC), hematocrit (Hct), and hemoglobin (Hb), etc. It is known that in normal conditions the absolute value of the hematocrit is about 3 times greater than the value of the hemoglobin, whereas in the presence of some anemias this balance can vary upward (hyperchromic anemia) or downward (hypochromic anemia).

Instrument:

Lambert-Beer's Law is an empirical relation which correlates the quantity of light absorbed by a means due to its chemical nature, its concentration and the thickness of the means passed through.

In photometers (spectro-photometers, colorimeters, turbidimeters), when a beam of light (mono- or poly-chromatic) with an intensity "$I_0$" passes through a layer with a thickness "L" of the analyzed sample to be measured, in this case the blood sample, a part of it is absorbed by the means itself and a part is transmitted with a residual intensity "$I_1$". The ratio between the light intensity transmitted through the means and the light intensity emitted is called Transmittance, and the formula which expresses it is the following:

$$T = I_1 / I_0$$

When T is equal to 100%, this means that the light is passing through a transparent sample.

Another parameter used is Absorbance, which represents the quantity of light absorbed by the sample analyzed and is expressed by the formula:

$$A = \log(1/T)*L = \log(I_0/I_1)*L$$

where L is the length of the optical path (thickness) which is conventionally equal to 1, equivalent to 1 centimeter.

This measurement directly expresses the concentration of the sample, when the latter is in a specific and suitable concentration.

In fact, the maximum accuracy of the photometer is expressed with a value equal to 36.8% of transmittance and, operatively, between 20 and 80% of cases, the relative error is contained within $\pm$ 2%.

In order to be able to use the instrument in this optimum measurement range, the analytical sample is suitably diluted, so as to be adapted to the measuring means.

Since the measurement of ESR can be determined only on the primary, native blood sample, of which no dilutions can be made, in order to adapt it to the measuring instrument, in the present invention a particular size of the optical path (L) was studied in order to make the use of the instrument come within said optimum measuring range.

The use of an optical path (L) of 0.8 mm allowed to use the microphotometer of the instrument to detect ESR as described in EP'188 in the relative absorbance range 0.500 $\div$ 0.950, which corresponds to the experimental range found for the above-mentioned 362 samples of blood on which the measurement was made.

Method:

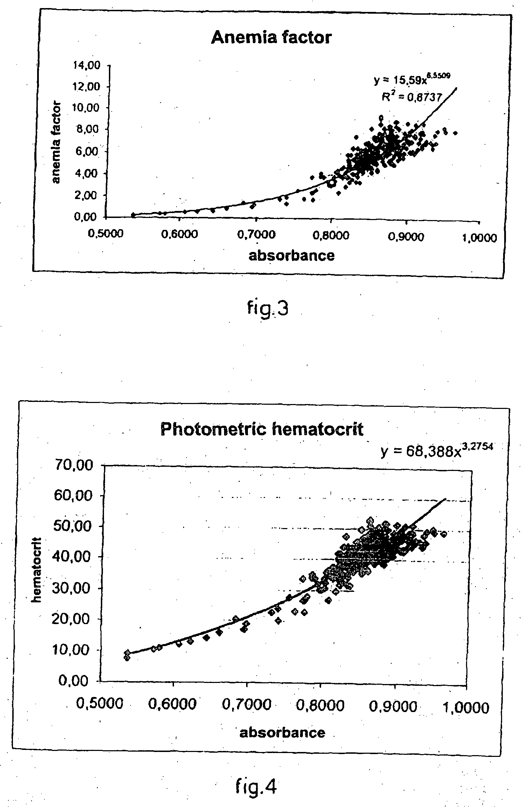

In consideration of the fact that this photometric measurement is mainly influenced by the number of red corpuscles, and therefore by the hematocrit, and by the relative hemoglobin value, a correspondence curve was constructed (fig. 3), by plotting all the values of absorbance $A_0$ experimentally obtained, which connects these values of the hemochrome with the value of absorbance measured by the apparatus shown in fig. 1.

As a result, a curve of correspondence of the values was obtained by interpolating the values of absorbance obtained, expressed in the numerical form describable with the equation:

$$Y = 15.59x^{6.5509},$$

with a correlation $R^2 = 0.8737$.

By comparing the curve obtained above, using the measurement of absorbance, with the curve resulting from the analytic measurement of the hematocrit values obtained, as we said, with the Coulter MAXM corpuscle-counter (see fig. 3), it was surprisingly possible to find, as will be seen from the table of comparison supplied hereafter, that the measurement of optical absorbance, obtained with said photometric instrument, allows to provide sufficiently coherent and reliable anemia values.

In other words, the Applicant has found that the values of absorbance obtained with the above disclosed method allow to obtain an indication of an anemia factor which has been proved to furnish an indication of the state of anemia of a patient, and this has been confirmed by comparing the so obtained results with the values obtained by calculating the anemia with the measurement of hematocrit.

Considering the hematocrit value exclusively, a numerical form derives, expressable with the equation $Y = 68.388^{3.2754}$ with a correlation $R^2 = 0.8737$.

Experimentally, as can be seen in the graph in fig. 4, low values of hematocrit and/or hemoglobin, with values of absorbance measured by the photometer shown in fig. 1 comprised between 0.500 and 0.800, allow to construct a curve in which the dispersion of data is contained within a limited field of values.

The aforesaid calculation algorithms were applied and independent measurements were performed on the above-mentioned 362 blood samples, with the corpuscle-counter, to obtain the hematocrit and hemoglobin values, in order to construct the reference values, and with the apparatus shown in fig. 1 to obtain the corresponding values of absorbance.

Putting the two anemia factors at 4.5, the one obtained by processing the hematocrit and hemoglobin values with that obtained by applying the numerical formula $Y = 15.59x^{6.55}$ to the absorbance values, the following analytical performances were obtained.

| Comparison of data - Coulter MAXM instrument and processing Anemia factor | | | | |
|---|---|---|---|---|
| $Y=15.59x^{6.5509}$ | | | | |
| TEST1 | Coulter MAXM | Positive | | Negative |
| positive | a: | 44 | b: | 12 |
| negative | c: | 10 | d: | 296 |
| Positive<br>True Positive | | 44 | a | |
| False Negative | | 12 | b | |
| True Negative | | 296 | d | |
| False Total samples | | 10<br>362 | c<br>a+b+c+d | |
| Sensitivity (SE) | | 0,8148 | a/(a+c) | |
| Specificity (SP) | | 0,9610 | d/(b+d) | |
| Pred. value POS. (PVP) | | 0,7857 | a/(a+b) | |
| Pred. value NEG. (PVN) | | 0,9673 | d/(c+d) | |
| | | | | |
| PREVALENCE (real) | | 0,1492 | (a+c)/(a+b+c+d) | |
| PREVALENCE (apparent) | | 0,1547 | (a+b)/(a+b+c+d) | |
| | | | | |
| Efficiency % | | 0,9392 | | |

In other words, the Applicant has found that, to determine an anemia factor, a concordance of 93% (efficiency) was demonstrated between the results obtained by studying the absorbance curve with respect to those obtained by measuring the hematocrit, which obviously requires a much longer time, more complex instruments, and more sophisticated processing procedures.

The study implemented, based on the construction of an absorbance curve, therefore allows to evaluate an alarm level determined by the photometric measurement of said corpusculate part, the hematocrit and the hemoglobin, as an agent suitable to signal said "Anemia Factor".

Said "anemia factor" can also be considered an indicator of good functioning for normal corpuscle-counters, because its result used as a reference or control of the two data supplied separately, that is, hematocrit and hemoglobin, can detect, if different from the state indicated, structural or functional anomalies of the individual measuring channels.

The anemia factor, in the verifications made, has a value below which there is a confirmation of low values, either of hematocrit or of hemoglobin, in the final results, and therefore confirms its alarm function to confirm the clinical datum supplied by the individual measurements with the corpuscle-counter and shows a potential case history that cannot be shown only by the measurement of ESR using the Westergren method.

In the same way, the anemia factor can be used to signal an inverse clinical situation, that is, an increased hematocrit value, polycytemia.

Modifications and variants may be made to the invention as described heretofore, which come within the scope of protection of the claims that follow.

## Claims

1. Method to detect states of anemia present in a blood sample, **characterized in that** it provides the following steps:

   i) providing a plurality of blood samples;
   ii) performing a hemochrome examination of said plurality of blood samples to measure hematocrit values of the blood samples;
   iii) constructing a correspondence curve described by an equation, connecting hematocrit values of the hemochrome examination of step ii) with values of absorbance ($A_0$) measured for each of said blood samples, wherein said values of absorbance ($A_0$) are obtained by:

      iii.a) disposing each of said blood samples in a containing seating associated with an optical detection system comprising a photometer with at least a light emitter (16) and a mating light receiver (17),
      iii.b) causing the break-up of the rouleaux formed by means of an interruption in the state of rest of the each of the blood samples, measuring the optical absorbance, or quantity of light absorbed, from the blood sample during steps iii.a) and iii.b),
      iii.c) constructing an absorbance curve so as to obtain a syllectogram and measuring the value of absorbance ($A_0$) in correspondence with the temporal instant (P) of maximum break-up of said rouleaux;

   iv) obtaining a correlation between a value of absorbance ($A_0$) measured from a syllectogram performed on a blood sample of a patient and a possible presence of a state of anemia in the patient from which the blood sample has been taken.

2. Method as in claim 1, **characterized in that** said containing seating for the blood sample consists of a capillary tube (12) and said interruption of the state of rest is obtained by means of an interruption of the blood flow through said capillary tube (12).

3. Method as in claim 2, **characterized in that** said capillary tube (12) defines an optical path (thickness) equal to 0.8 mm, which allows a relative optical absorbance range comprised between 0.500 and 0.950.

4. Method as in claim 1, **characterized in that** said containing seating for the blood sample consists of a containing cell associated with an agitation system that can be selectively activated.

5. Method as in claim 1, **characterized in that** said containing seating consists of a container associated with a vibration system that can be selectively activated.

6. Method as in any claim hereinbefore, **characterized in that** said blood sample is undiluted native blood.

## Patentansprüche

1. Verfahren zum Nachweis von in einer Blutprobe vorliegenden anämischen Zuständen, **dadurch gekennzeichnet,**

**dass** es die folgenden Schritte bereitstellt:

i) Bereitstellen einer Vielzahl von Blutproben;

ii) Durchführen einer Hämochrom-Prüfung der Vielzahl von Blutproben, um Hämatokritwerte der Blutproben zu messen;

iii) Erstellen einer Korrespondenz-Kurve, die durch eine Gleichung beschrieben ist, die die Hämatokritwerte der Hämochrom-Prüfung von Schritt ii) mit den für jede der Blutproben gemessenen Absorptionswerten ($A_0$) verbindet, wobei die Absorptionswerte ($A_0$) erhalten werden durch:

iii.a) Anordnen jeder der Blutproben in einer Aufnahmehalterung, die mit einem optischen Detektionssystem, das ein Photometer mit mindestens einem Lichtsender (16) und einem passend Lichtempfänger (17) umfasst, verbunden ist,

iii.b) Veranlassen des Aufbrechens der gebildeten Rouleaux durch eine Unterbrechung des Ruhezustands jeder der Blutproben, Messen der optischen Absorption oder Quantität des absorbierten Lichts aus der Blutprobe während der Schritte iii.a) und iii.b),

iii.c) Erstellen einer Absorptionskurve, um ein Syllektogramm zu erhalten und Messen des Absorptionswertes ($A_0$) im Verhältnis zum Zeitpunkt (P) des maximalen Aufbrechens der Rouleaux;

iv) Erhalten einer Korrelation zwischen einem Absorptionswert ($A_0$), gemessen von einem Syllektogramm, das an einer Blutprobe eines Patienten durchgeführt wurde und einem möglichen Vorliegen eines anämischen Zustands in dem Patienten, von dem die Blutprobe genommen wurde.

2. Verfahren gemäß Anspruch 1, **dadurch gekennzeichnet, dass** die Aufnahmehalterung für die Blutprobe aus einem Kapillarrohr (12) besteht und die Unterbrechung des Ruhezustands durch eine Unterbrechung des Blutflusses durch das Kapillarrohr (12) erreicht wird.

3. Verfahren gemäß Anspruch 2, **dadurch gekennzeichnet, dass** das Kapillarrohr (12) einen optischen Weg (Dicke) von 0,8 mm definiert, der einen relativen optischen Absorptionsbereich, der zwischen 0,500 und 0,950 umfasst, ermöglicht.

4. Verfahren gemäß Anspruch 1, **dadurch gekennzeichnet, dass** die Aufnahmehalterung für die Blutprobe aus einer Haltezelle besteht, die mit einem Bewegungssystem verbunden ist, das selektiv aktiviert werden kann.

5. Verfahren gemäß Anspruch 1, **dadurch gekennzeichnet, dass** die Aufnahmehalterung aus einem Behälter besteht, der mit einem Vibrationssystem verbunden ist, dass selektiv aktiviert werden kann.

6. Verfahren gemäß einem der vorstehenden Ansprüche, **dadurch gekennzeichnet, dass** die Blutprobe unverdünntes, natives Blut ist.

**Revendications**

1. Procédé pour détecter des états d'anémie dans un échantillon de sang, **caractérisé en ce qu'**il comporte les étapes consistant à :

i) disposer d'une pluralité d'échantillons de sang ;

ii) effectuer un hémogramme sur ladite pluralité d'échantillons de sang pour mesurer les valeurs de l'hématocrite des échantillons de sang ;

iii) construire une courbe de correspondance décrite par une équation qui mette en corrélation les valeurs de l'hématocrite de l'hématogramme de l'étape ii) avec des valeurs d'absorbance ($A_0$) mesurées pour chacun desdits échantillons de sang, dans lequel lesdites valeurs d'absorbance ($A_0$) sont obtenues par les étapes consistant à :

iii.a) disposer chacun desdits échantillons de sang dans un support apte à les contenir, associé à un système de détection optique comprenant un photomètre avec au moins un émetteur de lumière (16) et un récepteur de lumière correspondant (17),

iii.b) désagréger des rouleaux constitués en interrompant l'état de repos de chacun des échantillons de sang, en mesurant l'absorbance optique ou la quantité de lumière absorbée dans l'échantillon de sang pendant les étapes iii.a) et iii.b),

iii.c) construire une courbe d'absorbance de façon à obtenir un syllectogramme et mesurer la valeur de l'absorbance ($A_0$) en fonction de l'instant (P) de désagrégation maximale desdits rouleaux;

iv) obtenir une corrélation entre une valeur d'absorbance ($A_0$) mesurée à partir d'un syllectogramme effectué sur un échantillon de sang d'un patient et la présence possible d'un état d'anémie dans le patient dont l'échantillon de sang a été prélevé.

**2.** Procédé selon la revendication 1, **caractérisé en ce que** ledit support contenant l'échantillon de sang est constitué d'un tube capillaire (12) et ladite interruption de l'état de repos est obtenue au moyen d'une interruption du débit sanguin par ledit tube capillaire (12).

**3.** Procédé selon la revendication 2, **caractérisé en ce que** ledit tube capillaire (12) définit un chemin optique (épaisseur) de 0,8 mm, ce qui permet d'obtenir une plage d'absorbance optique relative comprise entre 0,500 et 0,950.

**4.** Procédé selon la revendication 1, **caractérisé en ce que** ledit support contenant l'échantillon de sang est constituée d'une cellule associée à un système d'agitation qui peut être activé sélectivement.

**5.** Procédé selon la revendication 1, **caractérisé en ce que** ledit support est constituée d'un récipient avec un système de vibration qui peut être activé sélectivement.

**6.** Procédé selon n'importe laquelle des revendications précédentes, **caractérisé en ce que** ledit échantillon de sang est sang natif non dilué.

fig. 1

Transmittance

fig. 2

fig.3

fig.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1090188 A
- US 4352557 A
- EP 0638799 A
- EP 1098188 A